# EUROPÄISCHE PATENTSCHRIFT

(11) **EP 2 203 425 B1**
(45) Veröffentlichungstag und Bekanntmachung des Hinweises auf die Patenterteilung: **14.06.2017**
(21) Anmeldenummer: 08804676.8
(22) Anmeldetag: 24.09.2008
(51) Int. Cl.: C07D 213/70

(54) **Biozide Mischungen**
Biocidal mixtures
Mélanges biocides

(30) Priorität: 26.09.2007 EP 07117221
(43) Veröffentlichungstag der Anmeldung: 07.07.2010
(73) Patentinhaber: LANXESS Deutschland GmbH, 50569 Köln (DE)
(72) Erfinder: STRAETMANS, Udo, 22143 Hamburg (DE); STRAETMANS, Felix, 22143 Hamburg (DE)
(86) Internationale Anmeldenummer: PCT/EP2008/062771
(87) Internationale Veröffentlichungsnummer: WO 2009/043773

(56) Entgegenhaltungen:
- GB-A- 1 330 531
- US-A- 4 161 526
- US-A- 4 443 222

## Beschreibung

Vorliegende Erfindung betrifft biozide Mischungen enthaltend zumindest ein Biozid, das kein Polyamin ist und zumindest ein Polyamin, sowie die Verwendung der vorgenannten bioziden Mischungen im Materialschutz. Die Offenbarung betrifft weiterhin ein Verfahren zum Entfärben und/oder zum Verhindern von Verfärbungen von Pyrithion enthaltenden technischen Stoffen durch Polyamine.

Biozide Mischungen enthaltend zumindest ein Biozid und zumindest ein Polyamin sind prinzipiell bekannt.

So ist in DE 40 33 272 C1 eine Zusammensetzung enthaltend 1,2-Benzisothiazolin-3-on (BIT) und ein Amin wie beispielsweise Lauryldipropylentriamin beschrieben, die weiterhin als Lösungsmittel Wasser und mit Wasser vollständig mischbare Lösungsmittel wie z.B. Butyldiglykol beinhalten.

Aus GB 1 191 253 sind wässrige Lösungen von rohem BIT bekannt, die zur Verbesserung der Lagerstabliität mit zwei oder mehreren Aminverbindungen aus der Gruppe Diethanolamin, Triethanolamis, Diisopropanolamin, Triisopropanolamin und Morpholin bekannt.

Ferner sind aus GB 1 330 531 Mischungen bekannt, die BIT und Amine mit 2 bis 6 C-Atomen enthalten, wobei die Amine frei von Hydroxyl- und Ethergruppen sind. Solche Mischungen sollen eine erhöhte Stabilität gegen Ausflocken aufweisen.

Aus DE sind 36 099 39 lagerstabile, flüssige Zubereitungen aus BIT und ethoxylierten, tertiären Aminen bekannt, die weiterhin Wasser, mit Wasser mischbare Lösungsmittel wie Polyglykole sowie weitere Hilfsstoffe wie Komplexbildner und/oder Wasserenthärtungsmittel enthalten.

Letztlich sind aus EP 612 470 A Desinfektionsmittel bekannt, die spezielle Glycolether und mindestens ein sekundäres oder tertiäres von Hydroxygruppen freies Amin beinhalten. Vorgenannte Desinfektionsmittel sollen eine verkürzte Einwirkzeit bzw. verbesserte Wirksamkeit aufweisen.

Spezielle Biozide sind Pyrithione. Pyrithione sind bekannte antimikrobielle Stoffe, die in einer Vielzahl von Anwendungen zur Konservierung nützlich verwendet werden. Natrium-Pyrithion (CAS 3811-73-2) ist ein wasserlösliches Pyrithion-Salz mit sehr guten antimikrobiellen Eigenschaften und wird z.B. als Konservierungsmittel (Biozid) in technischen Prozessflüssigkeiten und Hilfsmitteln wie z.B. Metallbearbeitungsflüssigkeiten, Schmierstoffen, Bindemitteln, Klebstoffen, Dichtungsmitteln, Lederhilfsmitteln, Papierbeschichtungsmitteln und sogar kosmetischen Präparaten verwendet. Die Herstellung von Natriumpyrithion wurde erstmals in US 3,159,640 beschrieben.

Leider besitzt Natriumpyrithion wie weitere Salze, z.B. Zinkpyrithion die Eigenschaft mit Schwermetallen, vor allem Eisenionen, intensiv gefärbte (dunkelgrün, blau, schwarz) Verbindungen einzugehen. Selbst wenn diese nur in Spuren vorhanden sind, z.B. als Katalysatorreste in Chemikalien oder natürliche Begleitstoffe (Eisen) von Additiven für Farben und Lacke (etc. oben genannte). Diese Verfärbungen sind nicht nur aus ästhetischen Gründen nicht akzeptabel, sondern führen auch zu massiven technischen Problemen wie z.B. Wirkungsverlust.

Beispielsweise unterliegen Ölkonzentrate einer bestimmten Spezifikation bei der Farbe, z.B. braun. Ein schwarzes Produkt ist nicht verkäuflich.

Metallbearbeitungsflüssigkeiten werden normalerweise als weiße Emulsionen oder schwach gelblich gefärbte Lösungen verwendet. Dunkelblaue bis schwarze Flüssigkeiten, die Werkstücke und Maschinen schwarz einfärben, führen zu eklatanten finanziellen Verlusten. In der Vergangenheit hat es Versuche zur Lösung der Verfärbung gegeben, die alle zu mehr oder weniger starken Nebeneffekten führen. In diesen Fällen wird versucht die Schwermetalle, besonders Eisenionen, durch andere Metallionen zu verdrängen bzw. durch Komplexbildung zu beseitigen. (Die Reaktion von Fe-III-Ionen mit Pyrithion führt zu einem schwarzen Niederschlag und wird so dem System entzogen. Damit verbunden ist ein Verlust an biozider Wirkung.) In Metallbearbeitungsflüssigkeiten führt die Verwendung von Komplexbildnern zwar zur Entfärbung von Fe-Pyrithion, vermindert allerdings auch die Wasserhärte und führt fast immer zur unerwünschten Schaumbildung der Flüssigkeit und Schaum kann weder schmieren noch kühlen. Eine weitere Möglichkeit ist die Verwendung von Salzen, die mit Pyrithion zu ungefärbten Komplexsalzen führen, z.B. Zink-Salzen (US 4,161,526). Auch diese Verfahrensweise führt zwar zu einer Entfärbung, allerdings auch zu einer Verminderung der bioziden Wirkung des Pyrithions und zu unerwünschten Folgeschäden.

Vor dem Hintergrund des vorstehend aufgeführten Standes der Technik war es wünschenswert, weitere biozide Mischungen bereitzustellen, die sich einerseits durch einen verringerten Anteil an Bioziden bei zumindest vergleichbarer Wirksamkeit auszeichnen und die weiterhin, insbesondere bei Verwendung von Pyrithionen diese Wirksamkeit auch in Gegenwart von Metallionen beibehalten können.

Es wurden nun biozide Mischungen gefunden enthaltend
- zumindest ein Biozid, das kein aliphatisches, primäres Polyamin ist und
- zumindest ein aliphatisches, primäres Polyamin, das zumindest 5 Kohlenstoffatome aufweist und keine sekundären, tertiären oder quartären Aminogruppen trägt,
wobei solche bioziden Mischungen ausgenommen sind, die Diaminohexan und 1,2-Benzisothiazolin-3-on enthalten, wie sie in GB 1 330 531 beschrieben sind.

Unter dem Begriff "aliphatisches, primäres Polyamin" sind im Rahmen der Erfindung solche Verbindungen zu verstehen, die zumindest zwei primäre Aminogruppen besitzen, die an ein sp³-hybridisiertes Kohlenstoffatom gebunden sind. Typischerweise weisen solche Polyamine für sich genommen keine anwendbaren bioziden Eigenschaften auf.

Es sei an dieser Stelle angemerkt, dass der Rahmen der Erfindung alle beliebigen und möglichen Kombinationen der oben stehenden und im Folgenden aufgeführten, allgemeinen oder in Vorzugsbereichen genannten Komponenten, Wertebereiche bzw. Verfahrensparameter umfasst.

Als Biozide, die keine aliphatischen, primären Polyamine sind, kommen erfindungsgemäß beispielsweise Fungizide, Bakterizide oder Algizide bzw. Mischungen solcher Wirkstoffe zum Einsatz, bevorzugt Fungizide oder Bakterizide oder Mischungen solcher Wirkstoffe.

Fungizide bzw. Bakterizide sind beispielsweise geeignet:
Aldehyde wie Zimtaldehyd, Formaldehyd, Glutardialdehyd, β-Bromzimtaldehyd, o-Phthaldialdehyd;
Benzimidazole wie Carbendazim, Benomyl, Fuberidazole, Thiabendazol oder deren Salze;
Benzthiazole wie: 2-Mercaptobenzothiazol;
Benzthiophendioxide wie:Benzo[b]thiophen-S,S-dioxid-carbonsäurecyclohexylamid;
Benzamide wie: 2,6-Dichloro-N-(4-trifluoromethylbenzyl)-benzamid, Tecloftalam;
Chinoline wie: 8-Hydroxychinolin und deren Cu-Salze;
Dithiocarbamate wie Cufraneb, Ferban, Kalium-N-hydroxymethyl-N'-methyl-dithiobarbamat, Na- oder K-dimethyldithiocarbamat, Macozeb, Maneb, Metam, Metiram, Thiram, Zineb, Ziram;
Formaldehyd und formaldehydabspaltende Verbindungen wie Benzylalkoholmono-(poly)-hemiformal, 1,3-Bis(hydroxymethyl)-5,5-dimethy-limidazolidine-2,4-dione (DMDMH), Bisoxazolidine, n-Butanol-hemiformal, Cis 1-(3-Chlorallyl)-3,5,7-triaza-1-azoniaadamantane chlorid, 1-[1,3-Bis(hydroxymethyl-2,5-dioxoimidazolidin-4-yl]-1,3-bis(hydroxymethyl)urea, Dazomet, Dimethylol-harnstoff, 4,4-Dimethyl-oxazolidine, Ethylenglycol-hemiformal, 7-Ethylbicyclooxazolidine, Hexa-hydro-S-triazine, Hexamethylentetramin, N-Hydroxymethyl-N'-methylthioharnstoff, Methylenbis-morpholin, Natrium N-(Hydroxy-methyl)glycinat, N-Methylolchloracetamid, Oxazolidine, Paraformaldehyd, Taurolin, Tetrahydro-1,3-oxazin, N-(2-Hydroxypropyl)-amin-methanol, Tetramethylol-acetylen-diharnstoff (TMAD);
Imidazole wie Clotrimazol, Bifonazol, Climbazol, Econazol, Fenapamil, Imazalil, Isoconazol, Ketoconazol, Lombazol, Miconazol, Pefurazoat, Prochloraz, Triflumizol, Thiazolcar 1-Imidazolyl-1-(4'-chlorophenoxy)-3,3-dimethylbutan-2-on sowie deren Metallsalze und Säureaddukte;
Iodderivate wie Diiodmethyl-p-tolylsulfon, 3-Iod-2-propinyl-alkohol, 4-Chlorphenyl-3-iodpropargylformal, 3-Brom-2,3-diiod-2-propenylethylcarbamat, 2,3,3-Triiodallylalkohol, 3-Brom-2,3-diiod-2-propenylalkohol, 3-Iod-2-propinyl-n-hexylcarbamat, 3-Iod-2-propinyl-cyclohexylcarbamat, 3-Iod-2-propinyl-phenylcarbamat;
Isothiazolinone wie N-Methylisothiazolin-3-on, 5-Chlor-N-methylisothiazolin-3-on, 4,5-Trimethylenisothiazolinon, Benzisothiazolinon;
Methoxyacrylate oder Ähnliche wie Azoxystrobin, Dimoxystrobin, Fluoxastrobin, Kresoxim-methyl, Metominostrobin, Orysastrobin, Picoxystrobin, Pyraclostrobin, Trifloxystrobin, 2,4-dihydro-5-methoxy-2-methyl-4-[2-[[[[1-[3-(trifluoromethyl)phenyl]-ethylidene]amino]oxy]methyl]phenyl]-3H-1,2,4-triazol-3-one (CAS-Nr. 185336-79-2);
Mikrobizide mit aktivierter Halogengruppe wie Bronidox, 2-Brom-2-nitro-1,3-propandiol, 2-Brom-4'-hydroxy-acetophenon, 1-Brom-3-chlor-4,4,5,5-tetramethyl-2-imidazoldinone, β-Brom-β-nitrostyrol, Chloracetamid, Chloramin T, 1,3-Dibrom-4,4,5,5-tetrametyl-2-imidazoldinone, Dichloramin T, 3,4-Dichlor-(3H)-1,2-dithiol-3-on, 2,2-Dibrom-3-nitril-propionamid, 1,2-Dibrom-2,4-dicyanobutan, Halane, Halazone, Mucochlorsäure, Phenyl-(2-chlor-cyan-vinyl)sulfon, Phenyl-(1,2-dichlor-2-cyanvinyl)sulfon, Trichlorisocyanur-säure;
Morpholinderivate wie Aldimorph, Dimethomorph, Dodemorph, Falimorph, Fenpropidin, Fenpropimorph, Tridemorph, Trimorphamid und ihre arylsulfonsauren Salze, wie z.B. p-Toluolsulfonsäure und p-Dodecylphenylsulfonsäure;
Naphthalin-Derivate wie Terbinafin, Naftifin, Butenafin, 3-Chloro-7-(2-aza-2,7,7-trimethyl-oct-3-en-5-in);
Nitrile wie 2,4,5,6-Tetrachlorisophthalodinitril, Dinatrium-cyano-dithioimidocarbamat;
Phenole wie Tribromphenol, Tetrachlorphenol, 3-Methyl-4-chlorphenol, 3,5-Dimethyl-4-chlorphenol, Dichlorphen, 2-Benzyl-4-chlorphenol, Triclosan, Diclosan, Hexachlorophen, p-Hydroxybenzoesäuremethylester, p-Hydroxybenzoesäureethylester, p-Hydroxybenzoesäurepropylester, p-Hydroxybenzoesäurebutylester, p-Hydroxybenzoesäureoctylester ,o-Phenylphenol, m-Phenylphenol, p-Phenylphenol, 4-(2-tert.-Butyl-4-methyl-phenoxy)-phenol, 4-(2-Isopropyl-4-methyl-phenoxy)-phenol, 4-(2,4-Dimethyl-phenoxy)-phenol und deren Alkali- und Erdalkalimetallsalze;
Pyridine und Pyrimidine wie Ancymidol, Buthiobat, Fenarimol, Mepanipyrin, Nuarimol, Pyroxyfur, Triamirol; 1-Hydroxy-2-pyridinthion (und ihre Cu-, Na-, Fe-, Mn-, Zn-Salze), Tetrachlor-4-methylsulfonylpyridin, Pyrimethanol, Mepanipyrim, Dipyrithion, 1-Hydroxy-4-methyl-6-(2,4,4-trimethylpentyl)-2(1H)-pyridin und Pyrithion und seine Salze, wie insbesondere seine Zink- und ;
Quartäre Ammoniumverbindungen und Guanidine wie Benzalkoniumchlorid, Benzyldimethyltetradecylammoniumchlorid, Benzyldimethyldodecylammoniumchlorid, Dichlorbenzyldimethylalkylammoniumchlorid, Didecyldimethylammoniumchlorid, Dioctyldimethylammoniumchlorid, N-Hexadecyltrimethylammoniumchlorid, 1-Hexadecylpyridiniumchlorid, Iminoctadine-tris(albesilat);
Thiocyanate wie: Thiocyanatomethylthiobenzothiazol, Methylenbisthiocyanat;
Triazole wie Azaconazol, Azocyclotin, Bitertanol, Bromuconazol, Cyproconazol, Diclobutrazol, Difenoconazol, Diniconazol, Epoxyconazol, Etaconazol, Fenbuconazol, Fenchlorazol, Fenethanil, Fluquinconazol, Flusilazol, Flutriafol, Furconazol, Hexaconazol, Imibenconazol, Ipconazol, Isozofos, Myclobutanil, Metconazol, Paclobutrazol, Penconazol, Propioconazol, Prothioconazol, Simeoconazol, (±)-cis-1-(4-chlorphenyl)-2-(1H-1,2,4-triazol-1-yl)-cycloheptanol, 2-(1-tert-Butyl)-1-(2-chlorphenyl)-3-(1,2,4-triazol-1-yl)-propan-2-ol, Tebuconazol, Tetraconazol, Triadimefon, Triadimenol, Triapenthenol, Triflumizol, Triticonazol, Uniconazol sowie deren Metallsalze und Säureaddukte;
Succinat-Dehydrogenase-Inhibitoren wie Benodanil, Carboxim, Carboximsulfoxid, Cyclafluramid, Fenfuram, Flutanil, Furcarbanil, Furmecyclox, Mebenil, Mepronil, Methfuroxam, Metsulfovax, Nicobifen, Pyrocarbolid, Oxycarboxin, Shirlan, Seedvax;
Sulfenamide wie Dichlorfluanid, Tolylfluanid, Folpet, Fluorfolpet; Captan, Captofol;
sonstige Biozide wie Bethoxazin, 5-Hydroxy-2(5H)-furanon; 4,5-Benzdithiazolinon, 4,5-Trimethylendithiazolinon, N-(2-p-Chlorbenzoylethyl)-hexaminiumchlorid, 2-Oxo-2-(4-hydroxyphenyl)acethydroximsäure-chlorid, Tris-N-(cyclohexyldiazeniumdioxy)-aluminium, N-(Cyclohexyldiazeniumdioxy)-tributylzinn bzw. K-Salze, Bis-N-(cyclohexyldiazeniumdioxy)-kupfer, Iprovalicarb, Fenhexamid, Spiroxamin, Carpropamid, Diflumetorin, Quinoxyfen, Famoxadone, Polyoxorim, Acibenzolar-S-methyl, Furametpyr, Thifluzamide, Methalaxyl-M, Benthiavalicarb, Metrafenon, Cyflufenamid, Tiadinil, Teebaumöl, Phenoxyethanol,

Bevorzugte Fungizide bzw. Bakterizide sind:
Azaconazol, Bromuconazol, Cyproconazol, Dichlobutrazol, Diniconazol" Hexaconazol, Metaconazol, Penconazol, Propiconazol, Tebuconazol, Dichlofluanid, Tolylfluanid, Triadimefon, Fluorfolpet, Methfuroxam, N-Methyl-isothiazolin-3-on, 5-Chlor-N-methylisothiazolin-3-on, Dichlor-N-octylisothiazolinon, Mercaptobenzthiazol, Thiocyanatomethylthiobenzothiazol, Thiabendazol, Benzisothiazolinon, Pyrithion und seine Salze, 2-Brom-2-nitro-1,3-propandiol und o-Phenylphenol. Ganz besonders bevorzugt sind N-Methyl-isothiazolin-3-on, 5-Chlor-N-methylisothiazolin-3-on, Dichlor-N-octylisothiazolinon, Mercaptobenzthiazol, Thiocyanatomethylthiobenzothiazol, Thiabendazol, Benzisothiazolinon, Pyrithion bzw seine Alkalimetallsalze und o-Phenylphenol, wobei N-Methyl-isothiazolin-3-on, 5-Chlor-N-methylisothiazolin-3-on, Dichlor-N-octylisothiazolinon, Thiabendazol, Benzisothiazolinon und o-Phenylphenol noch weiter bevorzugt sind.

Als Algizide sind beispielsweise geeignet:
Acetochlor, Acifluorfen, Aclonifen, Acrolein, Alachlor, Alloxydim, Ametryn, Amidosulfuron, Amitrole, Ammonium sulfamate, Anilofos, Asulam, Atrazine, Azafenidin, Aziptrotryn, Azimsulfuron,
Benazolin, Benfluralin, Benfuresat, Bensulfuron, Bensulfid, Bentazon, Benzofencap, Benzthiazuron, Bifenox, Bispyribac, Bispyribac-Natrium, Borax, Bromacil, Bromobutide, Bromofenoxim, Bromoxynil, Butachlor, Butamifos, Butralin, Butylat, Bialaphos, Benzoyl-prop, Bromobutide, Butroxydim,
Carbetamid, Carfentrazone-ethyl, Carfenstrol, Chlomethoxyfen, Chloramben, Chlorbromuron, Chlorflurenol, Chloridazon, Chlorimuron, Chlornitrofen, Chloressigsäure, Chloransulam-methyl, Cinidon-ethyl, Chlorotoluron, Chloroxuron, Chlorpropham, Chlorsulfuron, Chlorthal, Chlorthiamid, Cinmethylin, Cinofulsuron, Clefoxydim, Clethodim, Clomazone, Chlomeprop, Clopyralid, Cyanamide, Cyanazine, Cycloat, Cycloxydim, Chloroxynil, Clodinafop-propargyl, Cumyluron, Clometoxyfen, Cyhalofop, Cyhalofop-butyl, Clopyrasuluron, Cyclosulfamuron,
Diclosulam, Dichlorprop, Dichlorprop-P, Diclofop, Diethatyl, Difenoxuron, Difenzoquat, Diflufenican, Diflufenzopyr, Dimefuron, Dimepiperate, Dimethachlor, Dimethipin, Dinitramine, Dinoseb, Dinoseb Acetate, Dinoterb, Diphenamid, Dipropetryn, Diquat, Dithiopyr, Diduron, DNOC, DSMA, 2,4-D, Daimuron, Dalapon, Dazomet, 2,4-DB, Desmedipham, Desmetryn, Dicamba, Dichlobenil, Dimethamid, Dithiopyr, Dimethametryn,
Eglinazin, Endothal, EPTC, Esprocarb, Ethalfluralin, Ethidimuron, Ethofumesat, Ethobenzanid, Ethoxyfen, Ethametsulfuron, Ethoxysulfuron,
Fenoxaprop, Fenoxaprop-P, Fenuron, Flamprop, Flamprop-M, Flazasulfuron, Fluazifop, Fluazifop-P, Fuenachlor, Fluchloralin, Flufenacet Flumeturon, Fluorocglycofen, Fluoronitrofen, Flupropanate, Flurenol, Fluridone, Flurochloridone, Fluroxypyr, Fomesafen, Fosamine, Fosametine, Flamprop-isopropyl, Flamprop-isopropyl-L, Flufenpyr, Flumiclorac-pentyl, Flumipropyn, Flumioxzim, Flurtamon, Flumioxzim, Flupyrsulfuron-methyl, Fluthiacet-methyl,
Glyphosate, Glufosinate-ammonium
Haloxyfop, Hexazinon,
Imazamethabenz, Isoproturon, Isoxaben, Isoxapyrifop, Imazapyr, Imazaquin, Imazethapyr, Ioxynil, Isopropalin, Imazosulfuron, Imazomox, Isoxaflutole, Imazapic,
Ketospiradox,
Lactofen, Lenacil, Linuron,
MCPA, MCPA-hydrazid, MCPA-thioethyl, MCPB, Mecoprop, Mecoprop-P, Mefenacet, Mefluidid, Mesosulfuron, Metam, Metamifop, Metamitron, Metazachlor, Methabenzthiazuron, Methazol, Methoroptryne, Methyldymron, Methylisothiocyanat, Metobromuron, Metoxuron, Metribuzin, Metsulfuron, Molinat, Monalid, Monolinuron, MSMA, Metolachlor, Metosulam, Metobenzuron,
Naproanilid, Napropamid, Naptalam, Neburon, Nicosulfuron, Norflurazon, Natriumchlorat,
Oxadiazon, Oxyfluorfen, Oxysulfuron, Orbencarb, Oryzalin, Oxadiargyl,
Propyzamid, Prosulfocarb, Pyrazolate, Pyrazolsulfuron, Pyrazoxyfen, Pyribenzoxim, Pyributicarb, Pyridat, Paraquat, Pebulat, Pendimethalin, Pentachlorophenol, Pentoxazon, Pentanochlor, Petroleumöle, Phenmedipham, Picloram, Piperophos, Pretilachlor, Primisulfuron, Prodiamine, Profoxydim, Prometryn, Propachlor, Propanil, Propaquizafob, Propazine, Propham, Propisochlor, Pyriminobac-methyl, Pelargonsäure, Pyrithiobac, Pyraflufen-ethyl,
Quinmerac, Quinocloamine, Quizalofop, Quizalofop-P, Quinchlorac,
Rimsulfuron
Sethoxydim, Sifuron, Simazine, Simetryn, Sulfosulfuron, Sulfometuron, Sulfentrazone, Sulcotrione, Sulfosate,
Teeröle, TCA, TCA-Natrium, Tebutam, Tebuthiuron, Terbacil, Terbumeton, Terbutylazine, Terbutryn, Thiazafluoron, Thifensulfuron, Thiobencarb, Thiocarbazil, Tralkoxydim, Triallate, Triasulfuron, Tribenuron, Triclopyr, Tridiphane, Trietazine, Trifluralin, Tycor, Thdiazimin, Thiazopyr, Triflusulfuron,
Vernolat.

Bevorzugte Algizide sind Triazinverbindungen, wie beispielsweise Terbutryn, Cybutryn, Propazin oder Terbuton, Harnstoffverbindungen, wie beispielsweise Diuron, Benzthiazuron, Methabenzthiazuron, Tebuthiuron, und Isoproturon, oder Uracile wie beispielsweise Terbacil.

In einer Variante der Erfindung enthalten die erfindungsgemäßen Mischungen weder Pyrithion noch Salze davon.

Die erfindungsgemäßen Mischungen enthalten weiterhin zumindest ein aliphatisches, primäres Polyamin, das zumindest 5 Kohlenstoffatome aufweist und keine sekundären, tertiären oder quartären Aminogruppen trägt.

Bevorzugte aliphatische, primäre Polyamine, die zumindest 5 Kohlenstoffatome aufweisen und keine sekundären, tertiären oder quartären Aminogruppen tragen sind:
Nicht-cyclische C₅-C₁₀ 1,w-Diamine wie 1,5-Pentandiamin, 1,5-Diamino-2-methylpentan, 1,7-Heptandiamin, 1,8-Octandiamin und 1,9 Nonandiamin;
cyclische C₆-C₁₀-Diamine wie 1,3-Bisaminomethyl-cyclohexan und 1,3-Xylylendiamin,
nicht-cyclische Polyetheramine wie Polyetheramine der Formeln I und II 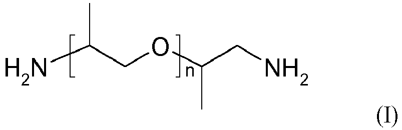 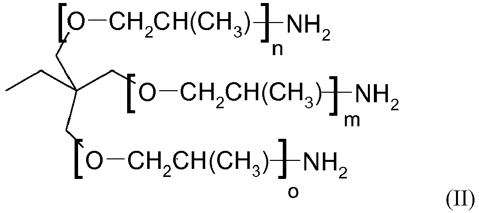
in denen
- n bzw. n, m und o: jeweils unabhängig voneinander für eine natürliche Zahl, bevorzugt für eine natürliche Zahl von 1 bis 10 und besonders bevorzugt 2 bis 5 steht
oder Mischungen der vorgenannten Polyamine.

Vorgenannte Amine der Formeln I und II sind beispielsweise als Polyetheramin D230 und Polyetheramin T403 kommerziell erhältlich.

Besonders bevorzugte aliphatische, primäre Polyamine, die zumindest 5 Kohlenstoffatome aufweisen und keine sekundären, tertiären oder quartären Aminogruppen tragen sind: 1,5-Diamino-2-methylpentan, 1,3-Bisaminomethyl-cyclohexan und 1,3-Xylylendiamin sowie Polyetheramine der Formeln I und II, mit den dort genannten Bedeutungen einschließlich ihrer Vorzugsbereiche sowie Mischungen der vorgenannten Polyamine.

Das Gewichtsverhältnis von Bioziden zu Polyaminen in den erfindungsgemäßen Mischungen kann beispielsweise 100:1 bis 1:100, bevorzugt 10:1 bis 1:10 und besonders bevorzugt 2:1 bis 1:5 betragen.

Gegebenenfalls können die erfindungsgemäßen Mischungen weiterhin saure Substanzen enthalten. Im Rahmen der Erfindung sind saure Substanzen Säuren mit einem Standard-pK_{A}-Wert von 2,0 bis 6,0 und saure Salze, die gemessen oder berechnet auf eine wässrige Skala und eine Konzentration von 1 mol/l einen pH-Wert von 2,0 bis 6,0 bei Standardtemperatur erzeugen.

Bevorzugte saure Substanzen sind: Ameisensäure, Essigsäure, Propionsäure, Buttersäure Bernsteinsäure, Milchsäure, Zitronensäure, Benzoesäure, Adipinsäure, Fumarsäure, Sorbinsäure, Salizylsäure, Sorbinsäure, Nonansäure.

Die erfindungsgemäßen Mischungen können weiterhin verschiedene Zusatzstoffe enthalten. Für die nachstehend genannten Zusatzstoffe besteht jeweils unabhängig voneinander auch die Möglichkeit, dass sie nicht enthalten sind. Mögliche Zusatzstoffe sind beispielsweise:
- Wasser. Die erfindungsgemäßen Mischungen können dabei beispielsweise 0,01 bis 98, vorzugsweise 5 bis 90 Gew-% an Wasser enthalten. Geeignete pH-Werte bei Standardbedingungen reichen in Abhängigkeit von der gewünschten Anwendung von 2 bis 11.
- Grenzflächenaktive Stoffe, wie beispielsweise Tenside. Tenside können beispielsweise nichtionische, kationische und amphotere Tenside, vorzugsweise anionische Tenside sein. Geeignete anionische Tenside sind beispielsweise Alkylsulfate, Alkylethersulfate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium-, Triethanolamin-Salze und Ammoniumsalze der vorgenannten Polyamine. Die Alkylethersulfate, Alkyletherphosphate und Alkylethercarboxylate können jeweils beispielsweise zwischen 1 bis 10 Ethylenoxid- oder Propylenoxid-Einheiten, bevorzugt 1 bis 3 Ethylenoxideinheiten aufweisen. Geeignet sind zum Beispiel Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat. Die erfindungsgemäßen Mischungen können dabei beispielsweise 0,01 bis 10, vorzugsweise 0,2 bis 8 Gew-% an grenzflächenaktiven Stoffen enthalten.
- Emulgatoren, wie beispielsweise Natrium-, Kalium-, Ammonium- und Ammoniumsalze der vorgenannten Polyamine von gradkettigen aliphatischen Carbonsäuren der Kettenlänge C₁₂-C₂₀, Natriumhydroxyoctadecansulfonat, Natrium-, Kalium-, Ammonium- und Ammoniumsalze der vorgenannten Polyamine von Hydroxyfettsäuren der Kettenlänge C₁₂-C ₂₀ und deren Sulfierungs-bzw. Acetylierungsprodukte, Alkylsulfate, auch als Triethanolaminsalze, Alkyl-(C₁₀-C₂₀)-sulfonate, Alkyl(C₁₀-C₂₀)-arylsulfonate, Dimethyldialkyl(C₈-C₁₈)-ammoniumchlorid, Acyl-, Alkyl-, Oleyl-und Alkylaryloxethylate und ihre Sulfierungsprodukte, Alkalisalze der Sulfobernsteinsäureester mit aliphatischen gesättigten einwertigen Alkoholen der Kettenlänge C₄-C₁₆, Sulfobernsteinsäure-4-Ester mit Polyethylenglykolethern von einwertigen aliphatischen Alkoholen der Kettenlänge C₁₀-C₁₂ (Di-Natriumsalz), Sulfobernsteinsäure-4-Ester mit Polyethylenglykolnonylphenylether (Di-Natriumsalz), Sulfobernsteinsäure bis-cyclohexylester (Natriumsalz), Ligninsulfonsäure sowie deren Calcium-, Magnesium-, Natrium-und Ammoniumsalze, Polyoxyethylensorbitanmonooleat mit 20 Ethylenoxidgruppen, Harzsäuren, hydrierte und dehydrierte Harzsäuren sowie deren Alkalisalze, dodecyliertes Diphenyletherdisulfonsaures Natrium sowie Copolymere aus Ethylenoxyd und Propylenoxyd mit einem Mindestgehalt von 10 Gew.-% Ethylenoxid. Vorzugsweise werden als Emulgatoren verwendet: Natriumlaurylsulfat, Natriumlaurylethersulfat, ethoxyliert (3 Ethylenoxidgruppen); die Polyethylenglykol(4-20)ether des Oleylalkohols sowie die Polyethenoxid-(4-14)ether von Nonylphenol. Die erfindungsgemäßen Mischungen können dabei beispielsweise 0,01 bis 15, vorzugsweise 0,2 bis 8, besonders bevorzugt 0,5 bis 6 und ganz besonders bevorzugt 1 bis 5 Gew.-% an Emulgatoren enthalten.
- Dispergiermittel, wie beispielsweise Alkylphenolpolyglycolether. Die erfindungsgemäßen Mittel können dabei beispielsweise 0,01 bis 8, vorzugsweise 0,1 bis 6, besonders bevorzugt 0,2 bis 5 und ganz besonders bevorzugt 0,2 bis 3 Gew.-% an Dispergiermitteln enthalten.
- Stabilisatoren, wie z.B. Cellulose und Cellulosederivate. Die erfindungsgemäßen Mittel können dabei beispielsweise 0,01 bis 6, vorzugsweise 00,1 bis 3, besonders bevorzugt 0,01 bis 2 und ganz besonders bevorzugt 0,01 bis 1 Gew.-% an Stabilisatoren enthalten.
- Spreitmittel, wie beispielsweise Isopropylmyristat Polyoxyethylennonylphenylether und Polyoxyethylenlaurylphenylether. Die erfindungsgemäßen Mischungen können dabei beispielsweise 0,01 bis 20, vorzugsweise 0,1 bis 10, besonders bevorzugt 0,1 bis 5 und ganz besonders bevorzugt 0,1 bis 2 Gew.-% an Spreitmitteln enthalten.
- Organische Lösungsmittel, wie beispielsweise ein- oder mehrwertige Alkohole, Polyether, Polyetheralkohole, Ester, Polyester, Polyesteralkohole, Ketone und Kohlenwasserstoffe. Die erfindungsgemäßen Mischungen können dabei beispielsweise 0,01 bis 95 an organischen Lösungsmittel enthalten.
- Duftstoffe und Farbstoffe, wie anorganische Pigmente, z.B. Eisenoxid, Titanoxid, Ferrocyanblau und organische Farbstoffe.
- Puffersubstanzen, Puffersysteme oder pH-Wert-Regulatoren. Die erfindungsgemäßen Mischungen können dabei beispielsweise jeweils 0,01 bis 10, vorzugsweise 0,1 bis 5, Gew.-% an Puffersubstanzen, Puffersystemen oder pH-Wert-Regulatoren enthalten Vorgenannte Polyamine können dabei ebenfalls als Bestandteil von Puffersystemen wirken, was sie zu wertvollen, multifunktionellen Komponenten in den erfindungsgemäßen Mischungen macht..

Von der Erfindung sind auch die erfindungsgemäßen Mischungen in beliebiger Formulierung umfasst. Bevorzugte Formulierungen sind Kapselsuspensionen (CS), Wasserlösliche Konzentrate (SL), Suspensionskonzentrate (SC) und Emulgierbare Konzentrate (EC), wobei Wasserlösliche Konzentrate (SL), Suspensionskonzentrate (SC) und Emulgierbare Konzentrate (EC) aufgrund der physikalischen Eigenschaften der Polyamine generell bevorzugt sind. Grundsätzlich hängen geeignete Formulierungsarten im Wesentlichen von den eingesetzten Mischungskomponenten und ihren physikalischen Eigenschaften ab. Da diese jedoch bekannt sind, ist es für den Fachmann gängige Praxis, in wenigen Versuchen weitere bevorzugte Formulierungsart zu ermitteln.

Der besondere Vorteil der erfindungsgemäßen Mischungen ist, dass der Gehalt an Polyaminen die Wirkung des oder der eingesetzten Biozidkomponenten verbessern können und anionische Tenside, die häufig Bestandteil von bioziden Formulierungen sind, nicht oder nur unwesentlich desaktivieren.

Die Erfindung betrifft daher weiterhin die Verwendung der erfindungsgemäßen Mischungen zum Schutz von technischen Materialien. Als technische Materialien kommen insbesondere Klebstoffe, Beton, Polymerdispersionen, Pigmentslurries, Tinten, Leime, Anstrichmittel, Beschichtungsmittel, Putze, Kühlschmiermittel und Wärmeüberleitungsflüssigkeiten bzw. funktionelle Flüssigkeiten zum Schutz der vorgenannten technischen Materialien in Frage. Ganz besonders bevorzugt sind Kühlschmiermittel und Klebstoffe.

Die Erfindung betrifft außerdem ein Verfahren zum Schutz von technischen Materialien vor Befall und/oder Zerstörung durch Mikroorganismen, das dadurch gekennzeichnet ist, dass man die erfindungsgemäßen Mischungen unverdünnt oder verdünnt auf den Mikroorganismus oder dessen Lebensraum einwirken lässt.

Die Erfindung betrifft außerdem technische Materialien, erhältlich durch Behandlung von technischen Materialien mit den erfindungsgemäßen Mischungen.

Als Mikroorganismen, die einen Abbau oder eine Veränderung der technischen Materialien bewirken können, seien beispielsweise Bakterien, Schimmel, Hefen, Algen und Schleimorganismen genannt. Schleimorganismen genannt. Vorzugsweise werden die erfindungsgemäßen Mischungen gegen Bakterien, Schimmel und Hefen eingesetzt.

Es seien beispielsweise Mikroorganismen der folgenden Gattung genannt:
Alternaria, wie Alternaria tenuis, Aspergillus, wie Aspergillus niger, Candids, wie candida albicans, Geotrichum wie Geotrichum candidum, Rhodotorula wie Rhodotorula rubra, saccharomyes wie Saccharomyces cerevisiae, Chaetomium, wie Chaetomium globosum,Coniophora, wie Coniophora puetana, Fusarium wie Fusarium solani, Lentinus, wie Lentinus tigrinus, Paecilomyces wie Paecilomyces variotti, Penicillium, wie Penicillium glaucum, Polyporus, wie Polyporus versicolor, Aureobasidium, wie Aureobasidium pullulans, Sclerophoma, wie Sclerophoma pityophila, Trichoderma, wie Trichoderma viride, Alcaligenes, wie Alcaligenes faecalis, Bacillus wie Bacillus subtilis, Escherichia, wie Escherichia coli, Proteus wie Proteus vulgaris, Pseudomonas, wie Pseudomonas aeruginosa und Pseudomonas fluorescens, Staphylococcus, wie Staphylococcus aureus.

Die Aufwandmenge der erfindungsgemäßen Mischungen richtet sich nach der Art und dem Vorkommen der zu bekämpfenden Mikroorganismen sowie nach der Zusammensetzung des zu schützenden Materials. Die optimale Einsatzmenge kann durch Testreihen einfach und in einer dem Fachmann hinlänglich bekannten Weise ermittelt werden.

Die Offenbarung betrifft biozide Mischungen enthaltend
- zumindest Pyrithion oder ein Salz davon
- gegebenenfalls zumindest ein weiteres Biozid, das kein aliphatisches, primäres Polyamin ist und
- zumindest ein Polyamin

Polyamine sind alle Verbindungen die zumindest zwei Aminogruppen enthalten. Bevorzugte Polyamine sind solche der Formeln III und IV 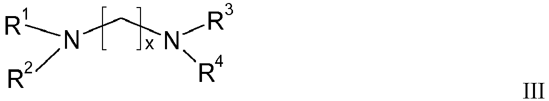 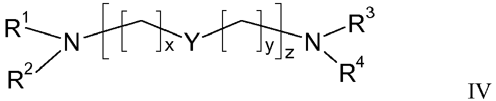 worin
- R¹, R², R³ und R⁴: für Wasserstoff, gesättigtes oder ungesättigtes, verzweigtes oder lineares C₁-C₁₀-Alkyl, Cycloalkyl, Ethoxylate, Propoxylate, Alkylaryl oder Heterocyclyl stehen kann
- Y: für O, NH, NR¹, R¹-C=C-R², Cycloalkyl, Ethoxyl, Propoxyl oder CH₂ stehen kann
- x, y und z: für eine natürliche Zahl N stehen

Beispiele für Polyamine der Formeln III und IV sind Diethylentriamin (CAS 111-40-0), Bis-(3-aminopropyl)ether (CAS 2579-20-6), 1,3-Bis(aminomethyl)cyclohexan (CAS 2579-20-6), Polyetheramin (CAS 39423-51-3), Diethylaminoethylamin (CAS 100-36-7), Diethylamino-propylamin (CAS 104-78-9), N-Aminopropylpiperidin und N-Aminopropylmorpholin (CAS 123-00-2.

Weitere bevorzugte Polyamine sind aliphatische, primäre Polyamine, die zumindest 5 Kohlenstoffatome aufweisen und keine sekundären, tertiären oder quartären Aminogruppen tragen, wobei die gleichen Bereiche und Vorzugsbereiche gelten wie sie oben genannt wurden.

Generell gelten für die Pyrithion enthaltenden Mischungen bezüglich der weiteren Inhaltsstoffe wie weitere Biozide, Wasser, grenzflächenaktive Stoffe, Emulgatoren, Dispergiermittel, Stabilisatoren, Spreitmittel, Organische Lösungsmittel,. Duftstoffe und Farbstoffe, sowie Puffersubstanzen, Puffersysteme oder pH-Wert-Regulatoren die oben dargestellte Offenbarung einschließlich der dort genannten Bereiche und Vorzugsbereiche völlig analog.

Als weitere Biozide sind allerdings weiterhin Hexahydrotriazin (HHT), Methylenbisoxazolidin (MBO), Methylenbismorpholin (MBM), DMDM-Hydantoin,(DMDMHy), Tetrahydro-1,3,4,6-tetrakis (hydroxymethyl)imidazo(4,5-d)imidazol-2,5(1H,3H)-dion, Benzisothiazolinon (BIT),o-Phenylphenol (OPP), Iodpropinylbutylcarbamat (IPBC) und Mischungen davon besonders bevorzugt.

Die Offenbarung betrifft weiterhin Ölkonzentrate, die allgemein wie folgt aufgebaut sind und bei Raumtemperatur in der angegebenen Reihenfolge gemischt werden können:
- bis 70% Basisöl (naphthalbasisch, paraffinbasisch, z.B. Nynäs T22)
- 20% - 40% Emulgatorsystem/Korrosionsschutz (Petrolsulfonate, Kalium- oder Aminseifen, Nichtionische Tenside, C₁₂-C₁₈ Ethoxylate). Weitere Emulgatoren sind obenstehend genannt.
- bis 40% Lösungsvermittler/Wasser
- 0,01 -5,0%, bevorzugt 0,1% - 3% Natriumpyrithion
- 0,01% - 20% Polyamine und deren Derivate

Diese allgemeine Rezeptur zeigt die Kombination von Pyrithion bzw. seinen Salzen mit Polyaminen. Die notwendige Dosierung der Polyamine beträgt beispielsweise das 0,2 bis 10-fache der Konzentration wie Natriumpyrithion und liegt insbesondere in der ungefähr gleichen Konzentration. Die beschriebenen Polyamine sind nicht nur zur Verhinderung von Verfärbungen geeignet, sondern können auch Amine ersetzen, die für die in der Rezeptur angegebenen Aminseifen aufgewandt werden müssen. Die eingesetzten Polyamine können ebenfalls zum Emulgieren wie zum Korrosionsschutz beitragen. Die Dosierung der Polyamine kann also vorteilhaft im Emulgatorsystem/Korrosionsschutz eingesetzt werden. Die Verwendung der beschriebenen Polyamine beschränkt sich nicht nur auf hoch ölhaltige Kühlschmierstoffe, sondern ist universell über halbsynthetische Produkte bis zu klar wasserlöslichen Vollsythesen geeignet.

Von der Offenbarung ist daher die Verwendung von Polyaminen zum Entfärben und/oder zum Verhindern von durch Schwermetallionen, insbesondere Eisen-III-Ionen, hervorgerufener Verfärbungen von Pyrithion und/oder Salze desselben enthaltenden Stoffen, bevorzugt von Ölkonzentraten, insbesondere Kühlschmierstoffkonzentraten, Farben und Lacken, Klebstoffen, Dichtungsmitteln, Lederhilfsmitteln, Papierbeschichtungsmitteln und Kosmetika umfasst, sowie die Stoffe, gekennzeichnet durch einen Anteil von einem oder mehreren Polyamin(en) und/oder deren Derivaten selbst.

Weiterhin umfasst die Offenbarung ein Verfahren zum Entfärben und/oder zum Verhindern von durch Schwermetallionen, insbesondere Eisen-III-Ionen, hervorgerufenen Verfärbungen von Pyrithion und/oder Salze desselben enthaltenden Stoffen, durch die Zugabe oder das Vorsehen eines Anteils von einem oder mehreren Polyaminen und/oder deren Derivaten.

### Beispiele

### I Mikrobiologische Beispiele

### Konservierung einer Kühlschmierstoffemulsion

Untersucht wurde die konservierende Wirkung der erfindungsgemäßen Gemische in einer Kühlschmierstoffemulsion (5 % Kühlschmierstoff-Konzentrat auf Mineralölbasis / 95 % Wasser). Hierzu wurden konservierte Proben der Kühlschmierstoffemulsion im wöchentlichen Rhythmus wiederholt einer mikrobiologischen Belastung mit folgenden Mikroorganismen ausgesetzt:

| | |
|---|---|
| Bakterien: | Pseudomonas aeruginosa |
| | Pseudomonas fluorescens |
| | Pseudomonas oleovorans |
| | Pseudomonas rubescens |
| | Pseudomonas stutzeri |
| | Alcaligenes faecalis |
| | Citrobacter freundii |
| | Corynebacterium sp |
| Hefen: | Rhodotorula rubra |
| Schimmelpilze: | Acremonium strictum |
| | Fusarium solani |
| | Geotrichum candidum |

Die Zugabe des Bakteriengemischs erfolgte jeweils getrennt von den Hefen/Schimmelpilzen. Die maximale Versuchsdauer betrug 10 Wochen (= 10 Kontaminationen/Impfzyklen), sofern bei der wöchentlichen Keimzahlbestimmung (jeweils 7 Tage nach der Kontamination) die folgenden Grenzwerte eingehalten wurden:
Bakterien < 10 ⁶ KBE/g
Hefen/ Schimmelpilze < 10 ³⁻⁴ KBE/g
Bei 2-maliger aufeinander folgender Überschreitung der genannten Werte wurde der Versuch angebrochen.

### Beispiel 1a): zum Vergleich

Versuch mit Benzisothiazolinon als Einzelkomponente in einer Konzentration von 0,05 % Benzisothiazolinon (= 500 ppm bzw. 2.500 ppm einer handelsüblichen 20 Gew%-igen Zubereitung) bezogen auf die Masse an Kühlschmierstoffemulsion.

Anzahl Wochen, in denen das Mikroorganismenwachstum unterhalb der Grenzwerte lag:

| | |
|---|---|
| Bakterien = | 1 Woche (von max. 10 Wochen) |
| Schimmelpilze = | 1 Woche (von max. 10 Wochen) |
| Hefen = | 7 Wochen (von max. 10 Wochen) |

### Beispiel 1b): erfindungsgemäß

Versuch mit erfindungsgemäßer Mischung enthaltend 20 Gew.% Benzisothiazolinon, 35 Gew.-% Polyetheramin D230, einem Amin der Formel I mit einem Molgewicht von ca. 230, 15 Gew.-% 1,5-Diamino-2-methylpentan und 30 Gew.-% Wasser. Vorstehend genannte, erfindungsgemäße Mischung wurde in einer Menge eingesetzt, so dass in der Kühlschmierstoffemulsion eine Wirkstoffmenge von 0,05 % (= 500 ppm) BIT resultierte:

**Anzahl Wochen, in denen das Mikroorganismenwachstum unterhalb der Grenzwerte lag:**

| | BIT/Amin | BIT |
|---|---|---|
| Bakterien = | 7 Wochen | 1 Woche |
| Schimmelpilze = | 1 Woche | 1 Woche |
| Hefen = | 10 Wochen | 7 Wochen |

### Resultat:

Die erfindungsgemäße Mischung zeigt eine gegenüber dem Einzelwirkstoff BIT deutlich verbesserte Gesamtwirkung, was insbesondere durch die wesentlich verbesserte Bakterienwirkung dokumentiert wird.

### Beispiel 2a): zum Vergleich

Versuch mit o-Phenylphenol (OPP) als Einzelkomponente mit einer OPP-Konzentration von 0,18 % OPP (= 1.800 ppm) bezogen auf die Masse an Kühlschmierstoffemulsion

**Anzahl Wochen, in denen das Mikroorganismenwachstum unterhalb der Grenzwerte lag:**

| | |
|---|---|
| Bakterien = | 2 Wochen (von max. 10 Wochen) |
| Schimmelpilze = | 10 Wochen (von max. 10 Wochen) |
| Hefen = | 10 Wochen (von max. 10 Wochen) |

### Beispiel 2b): erfindungsgemäß

Versuch mit erfindungsgemäßer Mischung enthaltend 55 Gew.% OPP (45 Gew.-% in Wasser), 30 Gew.-% Polyetheramin D230 und 15 Gew.-% 1,5-Diamino-2-methylpentan.

Vorstehend genannte, erfindungsgemäße Mischung wurde in einer Menge eingesetzt, so dass in der Kühlschmierstoffemulsion eine Wirkstoffmenge von 0,1 % (= 1.000 ppm) OPP resultierte:

**Anzahl Wochen, in denen das Mikroorganismenwachstum unterhalb der Grenzwerte lag:**

| | OPP/Amin | OPP |
|---|---|---|
| Schimmelpilze = | 10 Wochen | 10 Wochen |
| Hefen = | 10 Wochen | 10 Wochen |

### Resultat:

Die erfindungsgemäße Mischung zeigt eine gegenüber dem Einzelwirkstoff OPP deutlich verbesserte Gesamtwirkung, was insbesondere durch die wesentlich verbesserte Bakterienwirkung dokumentiert wird.

### II Beispiele zur Entfärbung (nicht erfindungsgemäß)

### Beispiel 3:

Ein vollsynthetischer Kühlschmierstoff (klare, farblose Flüssigkeit) bzw. dessen 5 %ige wässrige Lösung enthält 0,1 % eines Konservierungsmittels enthaltend 10 % Natriumpyrithion. In der klaren Lösung befinden sich also 100 ppm Natriumpyrithion. Dieser Lösung werden 0,01 g einer 5 %igen NH₄ Fe(III) (SO₄)₂ -Lösung zugetropft. Es entstand spontan eine intensiv tiefblau gefärbte Flüssigkeit. Nach Zugabe von 0,05 g Diethylaminoethylamin wurde die Lösung spontan entfärbt.

Für den gleichen Zweck eignen sich besonders:
Diethylaminoethylamin CAS 100-36-7, Diethylaminopropylamin CAS 104-78-9, N-Aminopropylpiperidin und, N-Aminopropylmorpholin CAS 123-00-2.

### Beispiel 4:

In einer klaren Lösung eines Kühlschmierstoffes befanden sich 100 ppm Natriumpyrithion. Dieser Mischung wurden 0,01 g einer 5 %igen NH₄ Fe(III) (SO₄)₂-Lösung zugetropft. Es entstand ummittelbar eine tiefblaue Lösung. Nach Zugabe von 0,05 g Diethylentriamin erhielt man sofort eine wasserklare Flüssigkeit.

Für den gleichen Zweck eignen sich besonders:
Diethylentriamin CAS 111-40-0, Bis-(3-aminopropyl)Ether CAS 2579-20-6, 1,3-Bis(aminomethyl)Cyclohexan CAS 2579-20-6, Polyetheramin CAS 39423-51-3

## Patentansprüche

1. Biozide Mischungen enthaltend
• zumindest ein Biozid, das kein aliphatisches, primäres Polyamin ist und
• zumindest ein aliphatisches, primäres Polyamin, das zumindest 5 Kohlenstoffatome aufweist und keine sekundären, tertiären oder quartären Aminogruppen trägt,
wobei solche bioziden Mischungen ausgenommen sind, die Diaminohexan und 1,2-Benzisothiazolin-3-on enthalten, wie sie in GB 1 330 531 beschrieben sind.

2. Biozide Mischungen nach Anspruch 1 **dadurch gekennzeichnet, dass** als Biozide, die keine aliphatischen, primären Polyamine sind Fungizide, Bakterizide und Algizide eingesetzt werden.

3. Biozide Mischungen nach Anspruch 1 oder 2, **dadurch gekennzeichnet, dass** als Biozide, die keine aliphatischen, primären Polyamine sind solche aus der Gruppe bestehend aus Azaconazol, Bromuconazol, Cyproconazol, Dichlobutrazol, Diniconazol,, Hexaconazol, Metaconazol, Penconazol, Propiconazol, Tebuconazol, Dichlofluanid, Tolylfluanid, Triadimefon, Fluorfolpet, Methfuroxam, N-Methyl-isothiazolin-3-on, 5-Chlor-N-methylisothiazolin-3-on, Dichlor-N-octylisothiazolinon, Mercaptobenzthiazol, Thiocyanatomethylthiobenzothiazol, Thiabendazol, Benzisothiazolinon, Pyrithion und seine Salze, 2-Brom-2-nitro-1,3-propandiol und o-Phenylphenol eingesetzt werden.

4. Biozide Mischungen nach einem der Ansprüche 1 bis 3, **dadurch gekennzeichnet, dass** sie weder Pyrithion noch Salze davon enthalten.

5. Biozide Mischungen nach einem der Ansprüche 1 bis 4, **dadurch gekennzeichnet, dass** als aliphatische, primäre Polyamine, die zumindest 5 Kohlenstoffatome aufweisen und keine sekundären, tertiären oder quartären Aminogruppen tragen solche eingesetzt werden, die ausgewählt sind aus der Gruppe bestehend aus 1,5-Pentandiamin, 1,5-Diamino-2-methylpentan, 1,7-Heptandiamin, 1,8-Octandiamin und 1,9 Nonandiamin, 1,3-Bisaminomethyl-cyclohexan, 1,3-Xylylendiamin und nicht-cyclischen Polyetheraminen der Formeln I und II 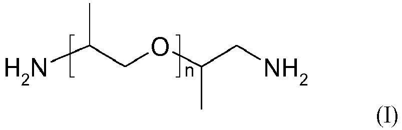 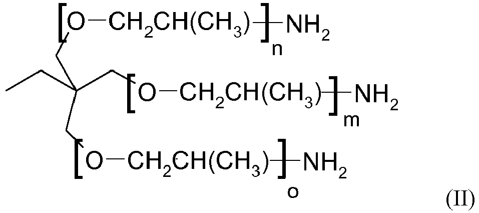 in denen
n bzw. n, m und o jeweils unabhängig voneinander für eine natürliche Zahl steht,
sowie Mischungen der vorstehenden Polyamine.

6. Biozide Mischungen nach einem der Ansprüche 1 bis 5, **dadurch gekennzeichnet, dass** das Gewichtsverhältnis von Bioziden zu Polyaminen 100:1 bis 1:100 beträgt.

7. Biozide Mischungen nach einem der Ansprüche 1 bis 6, **dadurch gekennzeichnet, dass** sie weiterhin saure Substanzen enthalten.

8. Biozide Mischungen nach einem der Ansprüche 1 bis 7, **dadurch gekennzeichnet, dass** sie als Kapselsuspensionen (CS), Wasserlösliche Konzentrate (SL), Suspensionskonzentrate (SC) und Emulgierbare Konzentrate (EC) formuliert sind.

9. Verwendung von bioziden Mischungen nach einem der Ansprüche 1 bis 8 zum Schutz von technischen Materialien.

10. Verwendung nach Anspruch 9, **dadurch gekennzeichnet, dass** technische Materialien Klebstoffe, Beton, Polymerdispersionen, Pigmentslurries, Tinten, Leime, Anstrichmittel, Beschichtungsmittel, Putze, Kühlschmiermittel und Wärmeüberleitungsflüssigkeiten bzw. funktionelle Flüssigkeiten zum Schutz der vorgenannten technischen Materialien sind.

11. Verfahren zum Schutz von technischen Materialien vor Befall und/oder Zerstörung durch Mikroorganismen, **dadurch gekennzeichnet ist, dass** man die bioziden Mischungen nach einem der Ansprüche 1 bis 8 unverdünnt oder verdünnt auf den Mikroorganismus oder dessen Lebensraum einwirken lässt.

12. Verfahren nach Anspruch 11, **dadurch gekennzeichnet, dass** Mikroorganismen Bakterien, Pilze, Hefen, Algen und Schleimorganismen sind.

13. Technische Materialien, erhältlich durch Behandlung von technischen Materialien mit bioziden Mischungen nach einem der Ansprüche 1 bis 8.

## Claims

1. Biocidal mixtures containing
• at least one biocide that is not an aliphatic primary polyamine and
• at least one aliphatic primary polyamine which has at least 5 carbon atoms and does not bear any secondary, tertiary or quaternary amino groups,
with those biocidal mixtures being excepted which contain diaminohexane and 1,2-benzisothiazolin-3-one, as described in GB 1 330 531.

2. Biocidal mixtures according to Claim 1, **characterized in that** the biocides that are not aliphatic primary polyamines are fungicides, bactericides and algicides.

3. Biocidal mixtures according to Claim 1 or 2, **characterized in that** the biocides that are not aliphatic primary polyamines are those from the group consisting of azaconazole, bromuconazole, cyproconazole, dichlobutrazole, diniconazole, hexaconazole, metaconazole, penconazole, propiconazole, tebuconazole, dichlofluanid, tolylfluanid, triadimefon, fluorfolpet, methfuroxam, N-methylisothiazolin-3-one, 5-chloro-N-methyl-isothiazolin-3-one, dichloro-N-octylisothiazolinone, mercaptobenzothiazole, thiocyanatomethylthiobenzothiazole, thiabendazole, benzisothiazolinone, pyrithione and salts thereof, 2-bromo-2-nitro-1,3-propanediol and o-phenylphenol.

4. Biocidal mixtures according to any one of Claims 1 to 3, **characterized in that** they do not contain either pyrithione or salts thereof.

5. Biocidal mixtures according to any one of Claims 1 to 4, **characterized in that** the aliphatic primary polyamines which have at least 5 carbon atoms and do not bear any secondary, tertiary or quaternary amino groups are those which are selected from the group consisting of 1,5-pentanediamine, 1,5-diamino-2-methylpentane, 1,7-heptanediamine, 1,8-octanediamine and 1,9-nonanediamine, 1,3-bisaminomethylcyclohexane, 1,3-xylylenediamine and noncyclic polyether amines of the formulae I and II 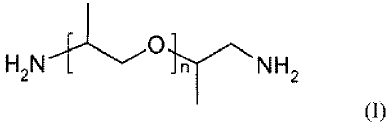 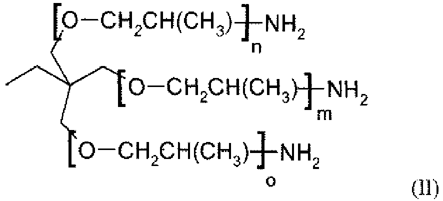 where
n or n, m and o in each case independently of one another is a natural number,
and also mixtures of the above polyamines.

6. Biocidal mixtures according to any one of Claims 1 to 5, **characterized in that** the weight ratio of biocides to polyamines is 100:1 to 1:100.

7. Biocidal mixtures according to any one of Claims 1 to 6, **characterized in that** they additionally contain acidic substances.

8. Biocidal mixtures according to any one of Claims 1 to 7, **characterized in that** they are formulated as capsule suspensions (CS), water-soluble concentrates (SL), suspension concentrates (SC) and emulsifiable concentrates (EC).

9. Use of biocidal mixtures according to any one of Claims 1 to 8 for protecting industrial materials.

10. Use according to Claim 9, **characterized in that** industrial materials are adhesives, concrete, polymer dispersions, pigment slurries, inks, sizes, paints, coatings, plasters, cooling lubricants and heat transfer liquids and/or functional liquids for protecting the abovementioned industrial materials.

11. Method for the protection of industrial materials against infestation and/or destruction by microorganisms, **characterized in that** the biocidal mixtures according to any one of Claims 1 to 8 are allowed to act undiluted or diluted on the microorganism or habitat thereof.

12. Method according to Claim 11, **characterized in that** microorganisms are bacteria, fungi, yeasts, algae and slime organisms.

13. Industrial materials obtainable by treating industrial materials with biocidal mixtures according to any one of Claims 1 to 8.

## Revendications

1. Mélanges biocides contenant :
- au moins un biocide, qui n'est pas une polyamine primaire aliphatique, et
- au moins une polyamine primaire aliphatique qui comprend au moins 5 atomes de carbone et ne porte pas de groupes amino secondaires, tertiaires ou quaternaires,
les mélanges biocides qui contiennent du diaminohexane et de la 1,2-benzisothiazolin-3-one étant exclus, comme décrits dans GB 1 330 531.

2. Mélanges biocides selon la revendication 1, **caractérisés en ce que** des fongicides, des bactéricides et des algicides sont utilisés en tant que biocides qui ne sont pas des polyamines primaires aliphatiques.

3. Mélanges biocides selon la revendication 1 ou 2, **caractérisés en ce qu'**en tant que biocides qui ne sont pas des polyamines primaires aliphatiques, celles du groupe constitué par l'azaconazole, le bromuconazole, le cyproconazole, le dichlobutrazole, le diniconazole, l'hexaconazole, le métaconazole, le penconazole, le propiconazole, le tébuconazole, le dichlofluanide, 1 tolylfluanide, le triadiméfon, le fluorfolpet, le méthfuroxam, la N-méthyl-isothiazolin-3-one, la 5-chloro-N-méthyl-isothiazolin-3-one, la dichloro-N-octylisothiazolinone, le mercaptobenzothiazole, le thiocyanatométhylthiobenzothiazole, le thiabendazole, la benzisothiazolinone, la pyrithione et ses sels, le 2-bromo-2-nitro-1,3-propanediol et l'o-phénylphénol sont utilisées.

4. Mélanges biocides selon l'une quelconque des revendications 1 à 3, **caractérisés en ce qu'**ils contiennent ni de la pyrithione, ni ses sels.

5. Mélanges biocides selon l'une quelconque des revendications 1 à 4, **caractérisés en ce qu'**en tant que polyamines primaires aliphatiques qui comprennent au moins 5 atomes de carbone et pas de groupes amino secondaires, tertiaires ou quaternaires, on utilise celles qui sont choisies dans le groupe constitué par la 1,5-pentanediamine, le 1,5-diamino-2-méthylpentane, la 1,7-heptanediamine, la 1,8-octanediamine et la 1,9-nonanediamine, le 1,3-bisaminométhyl-cyclohexane, la 1,3-xylylènediamine et les polyétheramines non cycliques des formules I et II 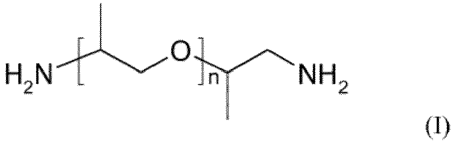 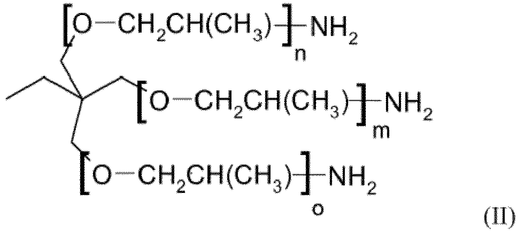 dans lesquelles
n ou n, m et o représentent chacun indépendamment les uns des autres un nombre naturel,
ainsi que les mélanges des polyamines précédentes.

6. Mélanges biocides selon l'une quelconque des revendications 1 à 5, **caractérisés en ce que** le rapport en poids entre les biocides et les polyamines est de 100:1 à 1:100.

7. Mélanges biocides selon l'une quelconque des revendications 1 à 6, **caractérisés en ce qu'**ils contiennent en outre des substances acides.

8. Mélanges biocides selon l'une quelconque des revendications 1 à 7, **caractérisés en ce qu'**ils sont formulés sous la forme de suspensions de capsules (CS), de concentrés solubles dans l'eau (SL), de concentrés de suspensions (SC) et de concentrés émulsifiables (EC).

9. Utilisation de mélanges biocides selon l'une quelconque des revendications 1 à 8 pour la protection de matériaux techniques.

10. Utilisation selon la revendication 9, **caractérisée en ce que** les matériaux techniques sont des adhésifs, du béton, des dispersions de polymères, des suspensions de pigment, des encres, des colles, des peintures, des agents de revêtement, des enduits, des huiles de refroidissement et des liquides de transfert de chaleur ou des liquides fonctionnels pour la protection des matériaux techniques susmentionnés.

11. Procédé de protection de matériaux techniques contre une infestation et/ou une destruction par des microorganismes, **caractérisé en ce que** les mélanges biocides selon l'une quelconque des revendications 1 à 8 sont laissés agir non dilués ou dilués sur les microorganismes ou leur habitat.

12. Procédé selon la revendication 11, **caractérisé en ce que** les microorganismes sont des bactéries, des champignons, des levures, des algues et des organismes mucilagineux.

13. Matériaux techniques, pouvant être obtenus par traitement de matériaux techniques avec des mélanges biocides selon l'une quelconque des revendications 1 à 8.
